# EUROPEAN PATENT SPECIFICATION

(11) **EP 0 979 237 B1**
(45) Date of publication and mention of the grant of the patent: **24.11.2004**
(21) Application number: 98908308.4
(22) Date of filing: 23.02.1998
(51) Int. Cl.: C07K 1/34, C07K 14/745, C12N 9/74, C07K 14/755

(54) **METHOD FOR REMOVING VIRUSES FROM PROTEIN SOLUTIONS BY NANOFILTRATION**
VERFAHREN ZUR ENTFERNUNG VON VIREN AUS PROTEINLÖSUNGEN MITTELS NANOFILTRATION
PROCEDE D'ELIMINATION PAR NANOFILTRATION DES VIRUS DES SOLUTIONS DE PROTEINE

(30) Priority: 24.02.1997 EP 97200533
(43) Date of publication of application: 16.02.2000
(73) Proprietor: Stichting Sanquin Bloedvoorziening, 1066 CX Amsterdam (NL)
(72) Inventor: HIEMSTRA, Harry, NL-1061 ST Amsterdam (NL); TER HART, Hendricus, Gerardus, Josephus, NL-1223 GG Hilversum (NL); PRINS-DE NIJS, Ingrid, Margaretha, Maria, NL-1749 EG Warmenhuizen (NL); HOEK, Pieter, Johannes, NL-2035 ST Haarlem (NL); OVER, Jan, NL-3533 EJ Utrecht (NL)
(74) Representative: Prins, Adrianus Willem, Mr. Ir.
(86) International application number: PCT/NL1998/000108
(87) International publication number: WO 1998/037086

(56) References cited:
- WO-A-96/00237
- WO-A-96/34947
- CHEMICAL ABSTRACTS, vol. 121, no. 24, 12 December 1994 Columbus, Ohio, US; abstract no. 286409, XP002067615 & M BURNOUF-RADOSEVICH ET AL.: "Nanofiltration, a new specific virus elimination method applied to high-purity factor IX and factor XI concentrates" VOX SANGUINIS , vol. 67, no. 2, 1994, pages 132-138,
- CHEMICAL ABSTRACTS, vol. 121, no. 26, 26 December 1994 Columbus, Ohio, US; abstract no. 308095, XP002067616 & M POULLE ET AL.: "Large-scale preparation of highly purified C1-inhibitor for therapeutic use" BLOOD COAGULATION FIBRYNOLYSIS, vol. 5, no. 4, 1994, pages 543-549,
- File Medline, abstract 97019642, 1997 XP002067614 & J RÖMISCH ET AL.: "Nanofiltration bei der Herstellung von Beriplex P/n: Erhöhung der Kapazität zur Viruseliminierung unter Beibehaltung der Produktqualität " BEITRÄGE ZUR INFUSIONSTHERAPIE UND TRANFUSIONSMEDIZIN, vol. 33, 1996, pages 220-224, cited in the application

## Description

### Field of the invention

This invention relates to methods to improve the viral safety of protein solutions, particularly blood products. More specifically, the invention relates to a method for removing viruses, in particular small, non-enveloped viruses, from a plasma derived product, such as Prothrombin Complex Concentrate (PCC).

### Background of the invention

Vitamin K-dependent plasma proteins, such as coagulation factors II, VII, IX and X and Proteins C and S, play an important role in the coagulation cascade. These factors isolated from plasma have been used since the early seventies to treat patients with a congenital deficiency of the afore-mentioned proteins (e.g. Hemophilia B) and patients with an acquired deficiency (e.g. unbalanced coumarin therapy). The product which contains the therapeutic vitamin K dependent proteins is also known as Prothrombin Complex Concentrate (PCC).

Widely used methods to improve the viral safety of blood products are chemical methods, such as the Solvent Detergent method, and heat treatment such as pasteurization or dry heat treatment. These methods are very effective for the removal or inactivation of enveloped viruses, such as Hepatitis B Virus (HBV), Hepatitis C Virus (HCV) and the Human Immunodeficiency Virus (HIV). However, these methods are less effective for non-enveloped viruses.

It is conventional to treat PCC in-process with virucidal chemicals (e.g., 0.3% TNBP and 1.0% Tween 80) to inactivate viruses. The viruses inactivated by such treatment are such enveloped viruses as Hepatitis B Virus, Hepatitis C Virus and Human Immunodeficiency Virus.

From the point of view from regulatory authorities there is a growing demand to prevent transmission by blood products of non-enveloped viruses, such as Hepatitis A virus (HAV) and Human Parvovirus B19 (HPV). These viruses typically show chemical and heat resistance and have a small size of 25 and 20 nm respectively. The existing viral inactivation methods have been largely unsuccessful in the inactivation of the Hepatitis A Virus and especially Human Parvovirus B19.

Nanofiltration (15 nm) as a virus removing technique has been applied with success for highly purified proteins with a molecular weight smaller than 150 kD (WO 96/00237, Pharmacia). Attempts to use nanofiltration in order to remove Hepatitis A Virus and Parvovirus B19 from a solution, such as Prothrombin Complex Concentrate, however, remained without success.

Prothrombin Complex Concentrate is a product of rather low purity which contains many proteins (estimated at about 20 to 30) with a molecular weight up to about 20,000 kD. The therapeutic components in Prothrombin Complex Concentrate have a molecular weight of only approximately 70 kD. It has been discovered that the Prothrombin Complex Concentrate cannot pass a nanofilter with a pore size of 15 nm because of the presence therein of these contaminants which are high molecular weight proteins. Passage of the therapeutic proteins through the nanofilter is effectively blocked by clogging of the membrane by high molecular weight proteins. Only 35 nm filtration is feasible for Prothrombin Complex Concentrate, but this type of filtration does not remove Hepatitis A Virus and Human Parvovirus B19 (J. Römisch et al., Beitr. Infusions-ther. Transfusionsmed. Basel, Karger, 1996, vol. 33, 220-224) see also M. Burnouf - Radosevich et al. Vox Sang. 67 : 132 - 138 (1994) and M. Poulle et al. Blood Coagulation and fibrinolysis 5: 543-549 (1994).

An object of this invention is to provide a method which overcomes this problem.

An object of the subject invention is to provide a method for the removal or inactivation of small viruses, such as in particular non-enveloped viruses like HAV and parvoviruses, from a complex mixture, in particular plasma derived products, such as Prothrombin Complex Concentrate.

Another object of this invention is to provide an effective method for removing small viruses, in particular non-enveloped viruses, by nanofiltration from a blood serum or plasma derived mixture containing one or more members of the group consisting of coagulation factors II, VII, IX and X and Proteins C and S, together with higher molecular weight proteins having a molecular weight of from about 200 kD, more particularly a molecular weight of from about 400 kD to about 20,000 kD.

### Summary of the invention

The invention realizes the above objects by providing a method in which a complex mixture, in particular Prothrombin Complex Concentrate, is subjected to a pretreatment which improves the filtration properties of the protein solution. Typically, the pretreatment comprises a filtration, such as filtration over a 150 kD membrane in a tangential flow method at a transmembrane pressure of less than 0.5 bar. After the pretreatment, the PCC can be filtered through a 15 nm nanofilter.

### Detailed description of the invention

More specifically, this invention relates to a method for removing viruses, in particular non-enveloped viruses, from a protein solution, comprising subjecting said mixture to a pretreatment which removes large proteins and subjecting the resulting product to nanofiltration which removes viruses. By applying the method of this invention to a protein solution which might be contaminated with (non-enveloped) viruses, it can be secured that the product obtained is substantially free of said viruses. So, the subject matter of this invention may be rephrased as a method for subjecting protein solutions to a treatment or processing which secures their substantial freedom of (non-enveloped) viruses.

The protein solution preferably is a blood derived protein solution, particularly a protein solution containing one or more proteins selected from the group consisting of coagulation factors II, VII, IX, X, Protein C, Protein S, albumin, antithrombin III, plasminogen, heparin cofactor II, Alpha-1-proteinase inhibitor, C1 inhibitor, transferrin, vitamin D-binding protein and immunoglobulin G. In a most preferred embodiment of the invention, said protein solution is Prothrombin Complex Concentrate.

Generally, the large proteins which are to be removed by the pretreatment will have a molecular weight larger than about 150 kD, more particularly a molecular weight of from about 400 to about 20,000 kD. The large proteins are generally selected from the group consisting of fibrinogen, fibronectin, immunoglobulin M, coagulation factors VIII, XIII, von Willebrand factor and its multimeric forms, inter-alpha-(trypsin)-inhibitor, alpha-2-macroglobulin, C1q complement protein, C4 complement protein, apolipoprotein(a), apolipoprotein B-100 and ferritin.

In a particularly preferred embodiment of the invention, the pretreatment comprises membrane filtration over a membrane having a cut-off value of between about 100 to about 250 kD, preferably about 150 kD. It is preferred that the membrane filtration is carried out in a tangential flow filtration mode at a transmembrane pressure of less than 0.5 bar, and that during filtration a buffer is added to the retentate and the filtration process is stopped when the amount of filtrate is about 4 to 6 times the amount of starting material.

The nanofiltration will generally be carried out over a nanofilter having a cut-off value of between about 10 to about 30 nm, preferably about 15 nm, in a dead-end filtration mode. The resulting nanofiltrate (i.e. the filtrate obtained in the nanofiltration) is preferably directly concentrated by dia- or ultrafiltration, preferably in a hemodialysis cartridge.

The viruses to be removed, if present, generally are non-enveloped viruses, in particular non-enveloped viruses having a diameter of 20 nm or more, such as, in particular, Hepatitis A Virus, parvoviruses such as Human Parvovirus B19 and Canine Parvovirus, and Encephalomyocarditis Virus.

The method may include a virus inactivation, preferably before said pretreatment, by treating the protein solution with detergents such as ionic and/or non-ionic detergents in the presence of di- or trialkyl phosphate compounds such as tri-n-butyl phosphate. Furthermore, the protein solution obtained may be lyophilized.

The present invention furthermore relates to a product, more in particular Prothrombin Complex Concentrate, which is substantially free of (non-enveloped) viruses and is obtainable with the method of this invention.

The subject invention, which comprises a combination of a prefiltration followed by a nanofiltration, is applicable for every therapeutic protein present in a solution with a complex composition. Such protein solution could be derived from blood plasma, body tissue, fermentation broths or other biological source materials. As stated before, the preferred starting material of this invention is PCC.

The high molecular weight contaminants that are typically removed by the prefiltration step have a molecular weight of at least 150 kD. Prefiltration removes, in the case of a blood derived protein solution, in particular proteins such as: fibrinogen, fibronectin, Immunoglobulin M, coagulation factor VIII, coagulation factor XIII, von Willebrand factor and its multimeric forms, inter-alpha-inhibitor (=inter-alpha-trypsin-inhibitor), Alpha-2-macroglobulin, C1q complement protein, C4 complement protein, apolipoprotein(a), apolipoprotein B-100 and ferritin.

Use of nanofiltration is limited to therapeutic proteins having a molecular weight up to 150 kD. In the case of blood derived protein solutions, nanofiltration is limited to such proteins as the coagulation factors II, VII, IX and X, protein S, protein C, albumin, antithrombin III, plasminogen, heparin cofactor II, Alpha-1-proteinase inhibitor, C1 inhibitor, transferrin, vitamin D-binding protein and immunoglobulin G.

The pretreatment and subsequent nanofiltration of PCC are preferably operated as follows:
A. The first step is filtration of PCC over a 150 kD membrane in a tangential flow method at a transmembrane pressure of less than 0.5 bar. The filtrate passing through the prefilter is collected. During filtration, buffer is added continuously to the retentate. The amount of buffer added is equal to the amount of filtrate obtained. Then, in a later stage of this filtration process, the addition of buffer is stopped. After completion of the prefiltration process the amount of filtrate preferably is 4 to 6 times the amount of starting material.
   During this prefiltration high molecular weight proteins in a molecular weight range of 400 kD or larger are removed from the PCC-solution. It was found that a similar prefilter with 300 kD membranes was not suitable for the essential removal of these high molecular weight components.
B. The second step is filtration of the prefiltered PCC over a 15 nm nanofilter in a dead-end filtration mode. During this process the 15 nm filtrate, i.e the flowthrough, is directly concentrated using hemodialysis cartridges in order to bring the coagulation factors to their desired concentration. The dead-end filtration mode is preferred to prevent a further dilution of the product and to prevent that characteristics of the product change, e.g. that the product becomes cloudy.

The filters used for prefiltration are available from Pall-Filtron (150 kD Omega low protein binding filters). The 15 nm nanofilter is available from Asahi Chemical Corp. Ltd (Tokyo, Japan). Suitable hemodialysis cartridges are available from several manufacturers, e.g. from Fresenius (HF 80).

The efficacy of the method for removal of non-enveloped viruses was studied by spiking the solution with a known amount of a model virus. For this study Canine Parvovirus (as a model for Human parvovirus B19) and Hepatitis A virus were used. These studies were performed on a scale that is 0.2 % of the production scale. The nanofiltration of PCC is capable of removing small non enveloped viruses to an extent of 5.0 to 6.0 on a 10-log scale.

The biochemical composition of nanofiltered PCC is only slightly altered when compared to unfiltered PCC. The level of coagulation factors and the concentration of excipients of freeze-dried nanofiltered PCC is similar to that of unfiltered freeze-dried PCC. In vivo and in vitro tests demonstrated that nanofiltration had no adverse effect on the product quality and characteristics.

The invention will be illustrated in more detail in the following examples.

### Example-1

This example illustrates an experiment performed with 1000 ml of Prothrombin Complex Concentrate derived from the conventional production. Prothrombin Complex Concentrate was obtained from human blood plasma and had been subjected, prior to the prefiltration, to a sequence of steps involving ion exchange chromatography, chemical virus inactivation with 0.3% (w/w) Tri-n-butylphosphate (TNBP) and 1% (w/w) Tween 80, ion exchange chromatography and concentration.

### Characteristics of Prothrombin Complex Concentrate:

Protein content: 25 mg per ml; Chemical composition: 10 mmol/l Na₃-citrate, 500 mmol/l NaCl; pH 7.0.

### Characteristics prefilter:

Manufacturer: Filtron; Type A: 200 kD polyethylene (PEAS) screen channel membrane; Effective surface area: 0.07 m².

### Composition Filtration buffer:

10 mmol/l Na₃-citrate, 500 mmol/l NaCl, pH 7.0.

Prothrombin Complex Concentrate was concentrated from 1000 ml to about 250 ml using the prefilter type A. Hereafter the volume of the retentate was kept at a constant level of 250 ml by continuously adding filtration buffer. After adding about 1000 ml of filtration buffer the addition of filtration buffer was stopped and concentration was continued. At the end of the process about 2000 ml of prefiltered Prothrombin Complex Concentrate was obtained.

The recovery of factor IX in the filtrate using the type A prefilter was about 73%. The average filtrate flow for the type A prefilter during the process was 9 liter per hour per m². This step was carried out at virtually no transmembrane pressure.

### Example 2

This example illustrates an experiment performed with 1000 ml of Prothrombin Complex Concentrate derived from the conventional production. Prothrombin Complex Concentrate was obtained from human blood plasma and was passed, prior to the prefiltration, through a sequence of treatments involving ion exchange chromatography, chemical virus inactivation with 0.3% (w/w) Tri-n-butylphosphate (TNBP) and 1% (w/w) Tween 80, ion exchange chromatography and concentration.

### Characteristics of Prothrombin Complex Concentrate:

Protein content: 25 mg per ml; Chemical composition: 10 mmol/l Na₃-citrate, 500 mmol/l NaCl; pH 7.0.

### Characteristics prefilter:

Manufacturer: Filtron; Type B: 300 kD polyethersulfon (PES) screen channel membrane; Effective surface area: 0.07 m².

### Composition Filtration buffer:

10 mmol/l Na₃-citrate, 500 mmol/l NaCl, pH 7.0.

Prothrombin Complex Concentrate was concentrated from 1000 ml to about 250 ml using the type B-prefilter mentioned above. Hereafter the volume of the retentate was kept at a constant level of 250 ml by continuously adding filtration buffer. After adding about 1000 ml of filtration buffer the addition was stopped and concentration was continued. At the end of the process about 2000 ml of prefiltered Prothrombin Complex Concentrate was obtained. This step was performed at virtually no transmembrane pressure.

The recovery of factor IX in the filtrate using the type B prefilter was 66%. The average filtrate flow for the type B prefilter during the process was 26 liter per hour per m² area.

### Example-3

This example illustrates an experiment performed with prefiltered Prothrombin Complex Concentrate derived from example 1.

### Characteristics Nanofilter:

Manufacturer: Asahi Chemical, Tokyo, Japan; Type: 15 nm; Material: hollow fibers of cuprammonium regenerated cellulose; Effective surface area: 0.03 m²; Type of filtration: dead end; Configuration: one single filter.

A total volume of 2000 ml of prefiltered Prothrombin Complex Concentrate was passed over the nanofilter in a dead-end filtration mode at a constant transmembrane pressure of 0.5 bar (recommendations of manufacturer). The filtrate flow decreased during the experiment from 14 to 10 liter per hour per m². The factor IX recovery over the nanofilter was 79% as compared to the prefiltered Prothrombin Complex Concentrate.

### Example 4

This example illustrates an experiment performed with prefiltered Prothrombin Complex Concentrate derived from example 2.

### Characteristics Nanofilter:

Manufacturer: Asahi Chemical, Tokyo, Japan; Type: 15 nm; Material: hollow fibers of cuprammonium regenerated cellulose; Effective surface area: 0.03 m²; Type of filtration: dead end; Configuration: one single filter.

Of the available 2000 ml prefiltered Prothrombin Complex Concentrate only about 1000 ml could be passed over the nanofilter in a dead-end filtration mode at a constant transmembrane pressure of 0.5 bar (recommendations of manufacturer). The filtrate flow decreased during the experiment from 14 to 4 liter per hour per m². This indicates clogging of the nanofilter. The factor IX recovery over the nanofilter was only 35% due to the premature termination of the filtration procedure.

### Example 5

Four identical experiments were performed with 1000 ml of Prothrombin Complex Concentrate derived from the conventional production. Prothrombin Complex Concentrate was obtained from human blood plasma and had been subjected, prior to the prefiltration, to subsequent treatments involving ion exchange chromatography, chemical virus inactivation with 0.3% (w/w) Tri-n-butylphosphate (TNBP) and 1% (w/w) Tween 80, ion exchange chromatography and finally concentration.

Characteristics of Prothrombin Complex Concentrate:
Protein content: 25 mg per ml; Chemical composition: 10 mmol/l Na₃-citrate, 150 mmol/l NaCl; pH 7.0.

### Characteristics prefilter:

Manufacturer: Filtron; Type A: 200 kD polyethersulfon (PES) screen channel membrane; Effective surface area: 0.07 m².

### Composition Filtration buffer:

10 mmol/l Na₃-citrate, 500 mmol/l NaCl, pH 7.0.

Prothrombin Complex Concentrate was concentrated in a stepwise fashion over the prefilter type A. The steps used, expressed as percentage of starting volume, were: 100 to 50%, 50 to 40%, 40 to 30% and 30 to 20%. After each concentration step the volume was kept constant by adding filtration buffer (about 250 ml per step). Only concentration was applied when the retentate was concentrated to about 20% of the starting volume. At the end of the process about 3000 ml of filtrate was obtained. This filtration step was performed at virtually no transmembrane pressure.

The average recovery of factor IX in the filtrate using the type A prefilter was 94%. The filtrate flow during the process decreased from 20 to 7 liter per hour per m².

### Example 6

This example illustrates an experiment performed with a pool of four prefiltered Prothrombin Complex Concentrates (about 12 liters) derived from example 5.

### Characteristics Nanofilter:

Manufacturer: Asahi Chemical, Tokyo, Japan; Type: 15 nm; Effective surface area: 0.03 m²; Type of filtration: dead end; Configuration: two nanofilters serially connected.

During nanofiltration the filtrate flow decreased linear from 16 to 11 liter per m² per hour. The recovery of factor IX was 96%.

### Example 7

This example illustrates an experiment performed with the nanofiltered Prothrombin Complex Concentrate derived from example 6.

### Characteristic of Hemodialysis cartridge:

Manufacturer: Fresenius, Bad Homburg, Germany; Type: Hemoflow HF 80; Effective surface area: 1.8 m²; Configuration: one single hemodialysis cartridge.

### Dialysis buffer:

Composition: 10 mmol/l Na₃-citrate, pH 7.0.

During the steps described in examples 5 and 6 the PCC was diluted approximately four to five fold. This diluted nanofiltered Prothrombin Complex Concentrate was concentrated using a hemodialysis hollow fiber cartridge. After concentration the same hemodialysis cartridge was used for decreasing the ionic strength of the solution by dialysis against dialysis buffer until an ionic strength of 16 mS per cm was reached. The recovery of factor IX in this step was 92%.

### Example 8

Four identical experiments were performed with 18 liter of Prothrombin Complex Concentrate derived from a conventional production. Prothrombin Complex Concentrate was obtained from human blood plasma and prior to prefiltration had been treated in subsequent steps involving ion exchange chromatography, chemical virus inactivation with 0.3% (w/w) Tri-n-butylphosphate (TNBP) and 1% (w/w) Tween 80, ion exchange chromatography and finally concentration.

### Characteristics of Prothrombin Complex Concentrate:

Protein content: 25 mg per ml; Chemical composition: 10 mmol/l Na₃-citrate, 500 mmol/l NaCl; pH 7.0.

### Characteristics prefilter:

Manufacturer: Filtron; Type A: 200 kD polyethersulfon (PES) screen channel membrane; Effective surface area: 3.6 m²; Flow mode: tangential.

### Characteristics Nanofilter:

Manufacturer: Asahi Chemical, Tokyo, Japan; Type: 15 nm; Effective surface area: 1 m²; Flow mode: dead end; Configuration: Two parallel sets of two serially connected 1 m² Planova filters.

### Characteristic of Hemodialysis cartridge:

Manufacturer: Fresenius, Bad Homburg, Germany, Hemoflow HF 80; Effective surface area: 1.8 m²; Configuration: two parallel mounted hemodialysis cartridges.

### Composition Filtration buffer:

10 mmol/l Na₃-citrate, 500 mmol/l NaCl, pH 7.0.

### Composition Dialysis buffer:

10 mmol/l Na₃-citrate, pH 7.0.

About 18 liters of Prothrombin Complex Concentrate was filtered over the prefilter with a transmembrane pressure of 0.3 bar. Prior to prefiltration the product was recirculated starting at a transmembrane pressure of 0.7 bar. Every five minutes the transmembrane pressure was decreased with 0.1 bar to a final operation transmembrane pressure of 0.3 bar. In this recirculation phase a gel layer was formed on the membrane which prevented 'throughput' of high molecular weight proteins. This resulted in about 60 liter prefiltered nanofiltrate which was subsequently passed over two parallel sets of two serially connected nanofilters. The filtrate derived from nanofiltration was concentrated in line four to six fold with two parallel mounted hemodialysis cartridges. Hereafter the ionic strength was reduced to 16 mS/cm by dialysis against dialysis buffer using the same hemodialysis cartridges. The recovery of factor IX for these four large scale experiments was about 72% as compared to the starting material.

### Example 9

Three identical experiments were performed with 20 liter of Prothrombin Complex Concentrate derived from a conventional production. Prothrombin Complex Concentrate was obtained from human blood plasma and prior to prefiltration had been treated in subsequent steps involving ion exchange chromatography, chemical virus inactivation with 0.3% (w/w) Tri-n-butylphosphate (TNBP) and 1% (w/w) Tween 80, ion exchange chromatography and finally concentration.

### Characteristics of Prothrombin Complex Concentrate:

Protein content: 15-25 mg per ml; Chemical composition: 10 mmol/l Na₃-citrate, 500 mmol/l NaCl; pH 7.0.

### Characteristics prefilter:

Manufacturer: Filtron; Type C: 150 kD polyethersulfon (PES) screen channel membrane; Effective surface area: 3.6 m²; Flow mode: tangential.

### Characteristics Nanofilter:

Manufacturer: Asahi Chemical, Tokyo, Japan; Type: 15 nm; Effective surface area: 1 m²; Flow mode: dead end; Configuration: one set of two serially connected 1 m² Planova filters.

### Characteristics of Hemodialysis cartridge:

Manufacturer: Fresenius, Bad Homburg, Germany, Hemoflow HF 80; Effective surface area: 1.8 m²; Configuration: two parallel mounted hemodialysis cartridges.

### Composition Filtration buffer:

10 mmol/l Na₃-citrate, 500 mmol/l NaCl, pH 7.0.

### Composition Dialysis buffer:

10 mmol/l Na₃-citrate, pH 7.0.

About 20 liters of Prothrombin Complex Concentrate was filtered over the prefilter as described in Example 8. This resulted in about 60 liter prefiltered nanofiltrate which was subsequently passed over two serially connected nanofilters. The filtrate derived from nanofiltration was concentrated in line four to six fold with two parallel mounted hemodialysis cartridges. Hereafter the ionic strength was reduced to 16 mS/cm by dialysis against dialysis buffer using the same hemodialysis cartridges. The recovery of factor IX for these three large scale experiments was about 50-70% as compared to the starting material.

### Example 10: Virus removal studies with Planova 15N

The virus removal capacity of nanofiltration was studied by spiking prefiltered Prothrombin Complex Concentrate with relevant viruses such as Human Immunodeficiency Virus (HIV) and Hepatitis A Virus (HAV), and model viruses such as Bovine Viral Diarrhoea Virus (BVDV), Pseudo Rabies Virus (PSR), Encephalomyocarditis Virus (EMC), and Canine Parvovirus (CPV). EMC, HAV and CPV are viruses belonging to the class of non-enveloped viruses. EMC, HAV and CPV have a diameter of 20 to 25 nm. CPV is considered to be a model virus for Human Parvovirus B19.

These spiking studies were performed on a scale of 100 ml (this is about 0.2% of the industrial scale) of prefiltered Prothrombin Complex Concentrate derived from example 1. The material spiked with the above mentioned viruses was passed over two serially connected Planova 15N filters with a surface area of 0.003 m² each. The virus titer was determined in the prefiltered material and in the nanofiltered Prothrombin Complex Concentrate. The virus removal is expressed as a logarithmic reduction factor (in 10 log-metric).

The results of the studies are given in table 1.

**Table 1**

| virus studied | Size (nm) | Lipid envelope | Physicochemical Resistance | Removal by nanofiltration over serially connected Planova filters (10 log-metric) |
|---|---|---|---|---|
| HIV | 80-120 | Yes | Low | > 7.0 |
| BVDV | 40 | Yes | Intermediate | > 5.9 |
| PSR | 120-200 | Yes | Intermediate | > 6.2 |
| EMC | 20 | No | Intermediate | > 7.3 |
| CPV | 20 | No | High | > 5.1 |
| HAV | 25 | No | High | > 5.9 |

These data show that nanofiltration is capable of reducing the virus load of the prefiltered Prothrombin Complex Concentrate with a factor of 10⁵ - 10⁷ for viruses with a size of 20 to 25 nm and above.

## Claims

1. A method for removing viruses from a protein-containing complex mixture derived from blood which mixture comprises
(i) proteins from the group consisting of the coagulation factors II, VII, IX, X, Protein C, Protein S, albumin, antithrombin III, plasminogen, heparin cofactor II, alpha-1-proteinase inhibitor, C1 inhibitor, transferrin, vitamin D binding protein and immunoglobulin G, and
(ii) proteins from the group consisting of fibrinogen, fibronectin, immunoglobulin M, coagulation factors VIII, XIII, von Willebrand factor and its multimeric forms, inter-alpha-(trypsin)-inhibitor, alpha-2-macroglobulin, C1q complement proteins, C4 complement protein, apolipoprotein (a), apolipoprotein B-100 and ferritin;
said method comprising
(a) subjecting the complex mixture to a pre-treatment which removes proteins from group (ii), said pre-treatment comprising a membrane filtration over a membrane having a cut-off value of between 100 to 250 kD, and
(b) subjecting the resulting mixture to a nanofiltration which removes viruses, said nanofiltration being carried out over a nanofilter having a cut-off value of between 10 and 30 nm.

2. A method according to claim 1, wherein the complex mixture contains proteins from group (i) having a molecular weight up to about 150 kD.

3. A method according to claim 1, wherein the complex mixture contains proteins from group (ii) having a molecular weight larger than about 150 kD.

4. A method according to claim 1, wherein the complex mixture contains proteins from group (ii) having a molecular weight larger than about 200 kD.

5. A method according to claim 1, wherein the complex mixture contains proteins from group (ii) having a molecular weight of from about 400 kD to about 20,000 kD.

6. A method according to claim 1, wherein said protein solution contains vitamin K dependent blood plasma proteins.

7. A method according to claim 1, wherein said protein solution is Prothrombin Complex Concentrate.

8. A method according to claim 1, wherein said pretreatment comprises membrane filtration over a membrane having a cut-off value of about 150 kD.

9. A method according to claim 1, wherein said pretreatment comprises membrane filtration carried out in a tangential flow filtration mode at a transmembrane pressure of less than 0.5 bar.

10. A method according to claim 9, wherein during filtration a buffer is added to the retentate and the filtration process is stopped when the amount of filtrate is about 4 to 6 times the amount of starting material.

11. A method according to claim 1, wherein said nanofiltration is carried out over a nanofilter having a cut-off value of about 15 nm.

12. A method according to claim 1, wherein said nanofiltration is carried out in a dead-end filtration mode.

13. A method according to claim 1, wherein the nanofiltrate is concentrated by dia- or ultrafiltration, preferably in a hemodialysis cartridge.

14. A method according to claim 1, wherein said viruses are non-enveloped viruses.

15. A method according to claim 1, wherein said viruses are non-enveloped viruses having a diameter of 20 nm or more.

16. A method according to claim 1, wherein said viruses are non-enveloped viruses selected from the group consisting of Hepatitis A Virus, parvoviruses such as Human Parvovirus B19 and Canine Parvovirus, and Encephalomyocarditis Virus.

17. A method according to claim 1, further including a virus inactivation, preferably before said pretreatment, by treating the protein solution with detergents such as ionic and/or non-ionic detergents in the presence of di- or trialkyl phosphate compounds such as tri-n-butyl phosphate.

18. A method according to claim 1, wherein the protein solution obtained is lyophilized.

## Patentansprüche

1. Verfahren zur Entfernung von Viren aus einer komplexen Protein-enthaltenden Mischung, die aus Blut stammt, wobei die Mischung umfasst
(i) Proteine aus der Gruppe, bestehend aus den Koagulationsfaktoren II, VII, IX, X, Protein C, Protein S, Albumin, Antithrombin III, Plasminogen, Heparin-Co-Faktor II, alpha-1-Proteinaseinhibitor, C1-Inhibitor, Transferrin, Vitamin D-Bindeprotein und Immunoglobulin G, und
(ii) Proteine aus der Gruppe, bestehend aus Fibrinogen, Fibronectin, Immunoglobulin M, Koagulationsfaktoren VIII, XIII, von-Willebrand-Faktor und seinen multimeren Formen, inter-alpha-(Trypsin)-Inhibitor, alpha-2-Macroglobulin, C1q-Komplementproteinen, C4-Komplementprotein, Apolipoprotein (a), Apolipoprotein B-100 und Ferritin;
wobei das genannte Verfahren umfasst
(a) Unterwerfen der komplexen Mischung einer Vorbehandlung, welche Proteine aus Gruppe (ii) entfernt, wobei genannte Vorbehandlung eine Membranfiltration über eine Membran mit einer Ausschlussgrenze von zwischen 100 und 250 kD umfasst, und
(b) Unterwerfen der resultierenden Mischung einer Nanofiltration, welche Viren entfernt, wobei genannte Nanofiltration über einen Nanofilter mit einer Ausschlussgrenze von zwischen 10 und 30 nm durchgeführt wird.

2. Verfahren nach Anspruch 1, wobei die komplexe Mischung Proteine aus Gruppe (i) mit einem Molekulargewicht bis zu ungefähr 150 kD enthält.

3. Verfahren nach Anspruch 1, wobei die komplexe Mischung Proteine aus Gruppe (ii) mit einem Molekulargewicht größer als ungefähr 150 kD enthält.

4. Verfahren nach Anspruch 1, wobei die komplexe Mischung Proteine aus Gruppe (ii) mit einem Molekulargewicht größer als ungefähr 200 kD enthält.

5. Verfahren nach Anspruch 1, wobei die komplexe Mischung Proteine aus Gruppe (ii) mit einem Molekulargewicht von ungefähr 400 kD bis ungefähr 20000 kD enthält.

6. Verfahren nach Anspruch 1, wobei genannte Proteinlösung Vitamin K-abhängige Blutplasmaproteine enthält.

7. Verfahren nach Anspruch 1, wobei genannte Proteinlösung Prothrombinkomplexkonzentrat ist.

8. Verfahren nach Anspruch 1, wobei genannte Vorbehandlung Membranfiltration über eine Membran mit einer Ausschlussgrenze von ungefähr 150 kD umfasst.

9. Verfahren nach Anspruch 1, wobei genannte Vorbehandlung eine Membranfiltration, durchgeführt in einem tangentialen Stromfiltrationsmodus ("tangential flow filtration mode") bei einem Transmembrandruck von weniger als 0,5 Bar umfasst.

10. Verfahren nach Anspruch 9, wobei während der Filtration ein Puffer zu dem Filterrückstand zugegeben wird und der Filtrationsprozess gestoppt wird, wenn die Menge des Filtrats ungefähr vier- bis sechsmal die Menge des Ausgangsmaterials beträgt.

11. Verfahren nach Anspruch 1, wobei genannte Nanofiltration über einen Nanofilter mit einer Ausschlussgrenze von ungefähr 15 nm durchgeführt wird.

12. Verfahren nach Anspruch 1, wobei genannte Nanofiltration im Endfiltrationsmodus ("dead-end filtration mode") durchgeführt wird.

13. Verfahren nach Anspruch 1, wobei das Nanofiltrat durch Dia- oder Ultrafiltration vorzugsweise in einer Hämodialysekassette ("hemodialyis cartridge") konzentriert wird.

14. Verfahren nach Anspruch 1, wobei genannte Viren Viren ohne Membranhülle ("non-enveloped") sind.

15. Verfahren nach Anspruch 1, wobei genannte Viren Viren ohne Membranhülle mit einem Durchmesser von 20 nm oder mehr sind.

16. Verfahren nach Anspruch 1, wobei genannte Viren Viren ohne Membranhülle, ausgewählt aus der Gruppe, bestehend aus Hepatitis-A-Virus, Parvoviren wie humanes Parvovirus B19 und Hundeparvovirus, und Encephalomyocarditisvirus sind.

17. Verfahren nach Anspruch 1, des Weiteren enthaltend eine Virusinaktivierung, vorzugsweise vor genannter Vorbehandlung durch das Behandeln der Proteinlösung mit Detergenzien wie ionischen und/oder nicht-ionischen Detergenzien in Anwesenheit von Di- oder Trialkylphosphatverbindungen wie tri-n-Butylphosphat.

18. Verfahren nach Anspruch 1, wobei die erhaltene Proteinlösung lyophilisiert wird.

## Revendications

1. Procédé d'élimination des virus d'un mélange complexe contenant des protéines et provenant du sang, ce mélange comprenant
(i) des protéines du groupe constitué par les facteurs de coagulation II, VII, IX, X, la protéine C, la protéine S, l'albumine, l'antithrombine III, le plasminogène, le cofacteur II de l'héparine, l'alpha-I-antitrypsine, l'inhibiteur de C1, la transferrine, la protéine de liaison de la vitamine D et l'immunoglobuline G, et
(ii) des protéines du groupe constitué par le fibrinogène, la fibronectine, l'immunoglobuline M, les facteurs de coagulation VIII, XIII, le facteur de von Willebrand et ses formes multimères, l'inhibiteur inter-alpha(trypsine), l'alpha-2-macroglobuline, les protéines du complément C1q, la protéine,du complément C4, l'apolipoprotéine (a), l'apolipoprotéine V-100 et la ferritine;
ledit procédé comprenant les étapes selon lesquelles
(a) on soumet le mélange complexe à un prétraitement qui sépare les protéines du groupe (ii), ledit prétraitement comprenant une filtration sur membrane sur une membrane dont le seuil de coupure a une valeur comprise entre 100 et 250 kD, et
(b) on soumet le mélange obtenu à une nanofiltration qui sépare les virus, ladite nanofiltration étant effectuée sur un nanofiltre dont le seuil de coupure a une valeur comprise entre 10 et 30 nm.

2. Procédé selon la revendication 1, dans lequel le mélange complexe contient des protéines du groupe (i) ayant une masse molaire allant jusqu'à environ 150 kD.

3. Procédé selon la revendication 1, dans lequel le mélange complexe contient des protéines du groupe (ii) ayant une masse molaire supérieure à environ 150 kD.

4. Procédé selon la revendication 1, dans lequel le mélange complexe contient des protéines du groupe (ii) ayant une masse molaire supérieure à environ 200 kD.

5. Procédé selon la revendication 1, dans lequel le mélange complexe contient des protéines du groupe (ii) ayant une masse molaire d'environ 400 kD à environ 20 000 kD.

6. Procédé selon la revendication 1, dans lequel ladite solution de protéines contient des protéines du plasma sanguin dépendantes de la vitamine K.

7. Procédé selon la revendication 1, dans lequel ladite solution de protéines est un concentré du complexe de prothrombine.

8. Procédé selon la revendication 1, dans lequel ledit prétraitement comprend une filtration sur membrane sur une membrane dont le seuil de coupure a une valeur d'environ 150 kD.

9. Procédé selon la revendication 1, dans lequel ledit prétraitement comprend une filtration sur membrane effectué dans un mode de filtration à flux tangentiel sous une pression transmembranaire inférieure à 0,5 bar.

10. Procédé selon la revendication 9, dans lequel, pendant la filtration, on ajoute un tampon au rétentat et on arrête le processus de filtration lorsque la quantité de filtrat est d'environ 4 à 6 fois la quantité de produit de départ.

11. Procédé selon la revendication 1, dans lequel ladite nanofiltration s'effectue sur un nanofiltre dont le seuil de coupure a une valeur d'environ 15 nm.

12. Procédé selon la revendication 1, dans lequel ladite nanofiltration s'effectue dans un mode de filtration frontale.

13. Procédé selon la revendication 1, dans lequel on concentre le nanofiltrat par dia- ou ultrafiltration, de préférence dans une cartouche d'hémodialyse.

14. Procédé selon la revendication 1, dans lequel lesdits virus sont des virus non enveloppés.

15. Procédé selon la revendication 1, dans lequel lesdits virus sont des virus non enveloppés ayant un diamètre d'au moins 20 nm.

16. Procédé selon la revendication 1, dans lequel lesdits virus sont des virus non enveloppés choisis dans le groupe constitué par le virus de l'hépatite A, des parvovirus comme le parvovirus humain B19 et le parvovirus canin, et le virus de l'encéphalomyocardite.

17. Procédé selon la revendication 1, comprenant en outre une inactivation des virus, de préférence avant ledit prétraitement, par traitement de la solution de protéines avec des détergents comme des détergents ioniques et/ou non ioniques en présence de phosphates de di- ou trialkyle comme le phosphate de tri-n-butyle.

18. Procédé selon la revendication 1, dans lequel on lyophilise la solution de protéines obtenue.
